(19) Europäisches Patentamt
European Patent Office
Office européen des brevets

(11) Veröffentlichungsnummer: 0 364 708 A1

# (12) EUROPÄISCHE PATENTANMELDUNG

(21) Anmeldenummer: 89116165.5

(22) Anmeldetag: 01.09.89

(51) Int. Cl.5: C07C 213/04 , C07C 215/08 , C07C 215/10 , C07C 215/42

(30) Priorität: 07.09.88 DE 3830351

(43) Veröffentlichungstag der Anmeldung: 25.04.90 Patentblatt 90/17

(84) Benannte Vertragsstaaten: AT BE CH DE ES FR GB GR IT LI LU NL SE

(71) Anmelder: Kali-Chemie Aktiengesellschaft
Postfach 220, Hans-Böckler-Allee 20
D-3000 Hannover 1(DE)

(72) Erfinder: Eicher, Johannes
Moorkamp 7
D-3008 Garbsen 5(DE)
Erfinder: Fazniewscy, Karlheinz
Im Gesenk 8
D-3160 Lehrte(DE)
Erfinder: Rudolph, Werner
Oderstrasse 38
D-3000 Hannover 71(DE)

(74) Vertreter: Lauer, Dieter, Dr.
c/o Kali-Chemie AG Postfach 220
Hans-Böckler-Allee 20
D-3000 Hannover 1(DE)

## (54) Verfahren zur Herstellung von alpha-Aminoalkanolen.

(57) Es wird die Herstellung von α-Aminoalkanolen, insbesondere 1-Aminopropan-2-, 3-diol, durch Umsetzen von entsprechenden Alkylenoxiden mit wäßriger Ammoniaklösung in Gegenwart von Ammoniumsalzen beschrieben.

EP 0 364 708 A1

Xerox Copy Centre

## Verfahren zur Herstellung von α-Aminoalkanolen

Die Erfindung betrifft die Herstellung von α-Aminoalkanolen, insbesondere 1-Aminopropan-2,3-diol, durch Umsetzen von entsprechenden Alkylenoxiden mit wäßriger Ammoniaklösung.

α-Aminoalkanole finden mannigfaltige Verwendung in der Industrie, z.B. als technische Ausgangsstoffe und Zwischenprodukte bei der Herstellung von Seifen, Tensiden, Arzneimitteln, Schädlingsbekämpfungsmitteln, Kunststoffen usw. So werden z.B. durch Umsetzung von α-Alkanolaminen mit Fettsäurederivaten Alkanolamide erhalten, deren Alkyläther als Seifen. Tenside oder Schmieröladditive eingesetzt werden. Alkanolamine werden auch als solche bei der Reinigung industrieller "Gase zur Abtrennung saurer Gasbestandteile und als u.a. die Benetzung fördernde Additive für Lösungen verwendet. 1-Aminopropan-2,3-diol wird beispielsweise als Zusatz in blasenfreien Tinten für Kugelschreiber, in photographischen Entwicklungslösungen oder in kosmetischen feuchtigkeitsbewahrenden Hautlotionen eingesetzt.

α-Aminoalkanole werden technisch im allgemeinen durch Umsetzung von Ammoniak mit entsprechenden Alkylenoxiden in wäßriger Lösung unter Druck bei erhöhter Temperatur hergestellt (s. Kirk-Othmer, Bd. 1, 1978, Seiten 950-952). Diese Verfahren haben den Nachteil, daß neben den α-Aminoalkanolen auch die Di- und Trialkanolamine entstehen. Wenn überwiegend α-Aminoalkanole erhalten werden sollen, muß ein großer Überschuß an Ammoniak eingesetzt werden. Das heißt, es muß unter hohem Druck oder in einem sehr großen Reaktionsvolumen, also sehr verdünnt in großen Mengen wäßriger Ammoniaklösung gearbeitet werden. Die Reaktoren müssen entsprechend groß ausgelegt werden. Die Abtrennung der Reaktionsprodukte und das Abdestillieren der großen Mengen an wäßriger Ammoniaklösung sind folglich sehr aufwendig und kostspielig.

Zur Herstellung von 1-Aminopropan-2,3-diol durch Umsetzen von Glycidol mit wäßriger konzentrierter Ammoniaklösung ohne Verwendung von Druck setzten z.B. Knorr et al (Ber. Deutsch. Chem. Ges. 32 - (1899), S. 750-793) und Baum et al (J. Org. Chem. 27 (1962), S. 2231-2233) ein Gewichtsteil Glycidol mit 100 Gew.-Teilen 25 %-igem wäßrigem Ammoniak um und erhielten nach destillativer Aufarbeitung je nach Destillationsbedingungen Ausbeuten zwischen 40 und 70 %. Das Gew.-Verhältnis Glycidol zu 25 %-iger wäßriger Ammoniaklösung von 1 : 100 entspricht einen Molverhältnis Glycidol zu Ammoniak von 1 : 109.

Um das Arbeiten in derart verdünnten Lösungen zu vermeiden, wurden Verfahren vorgeschlagen, welche Alkylenoxide, beispielsweise Glycidol, mit Ammoniak unter Druck in weitgehend wasserfreien Systemen umsetzen, wobei der Druck so hoch gewählt ist, daß das Ammoniak flüssig bleibt (vgl. DE-A1-30 14 098, DE-A1-30 14 129 und DE-A1-30 14 109). Bei einem derartigen Verfahren kann zwar mit einem geringeren Reaktionsvolumen gearbeitet werden, das Arbeiten mit flüssigem Ammoniak unter Druck erfordert jedoch ebenfalls einen erheblichen technischen Aufwand.

Aufgabe der Erfindung ist es, ein technisches Verfahren zur Herstellung von α-Aminoalkanolen, insbesondere von 1-Aminopropan-2,3-diol, durch Umsetzen entsprechender Alkylenoxide in wäßriger Ammoniaklösung zu finden, welches die Nachteile der vorstehend bekannten Verfahren vermeidet.

Es wurde nun ein Verfahren gefunden, in welchem Alkylenoxide mit wäßriger Ammoniaklösung druckfrei zu α-Alkanolaminen umgesetzt werden können, und bei welchem nur ein Bruchteil der bei den vorbekannten druckfreien Umsetzungen benötigten Mengen an wäßriger Ammoniaklösung eingesetzt werden muß.

Gegenstand der Erfindung ist ein Verfahren zur Herstellung von α-Aminoalkanolen durch Umsetzen von Alkylenoxiden mit wäßriger Ammoniaklösung, dadurch gekennzeichnet, daß man in mindestens 20 Gew.-%-iger wäßriger Ammoniaklösung lösliche oder dispergierbare Alkylenoxide der allgemeinen Formel II

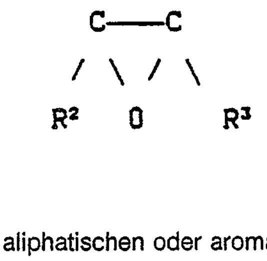

II

worin

$R^1$ Wasserstoff oder einen aliphatischen oder aromatischen Rest bedeutet,

$R^2$ Wasserstoff oder niederes Alkyl bedeutet,

$R^3$ Wasserstoff oder niederes Alkyl bedeutet, und
$R^4$ Wasserstoff oder niederes Alkyl bedeutet, oder
$R^4$ und $R^1$ gemeinsam mit den beiden Kohlenstoffatomen, an welche sie gebunden sind, eine carbocyclische Gruppe bilden,
mit mindestens 20 Gew.-%-iger wäßriger Ammoniaklösung in Gegenwart einer dem Alkylenoxid mindestens äquimolaren Menge eines in der mindestens 20 gew.-%-igen wäßrigen Ammoniaklösung löslichen Ammoniumsalzes umsetzt, und das gebildete α-Aminoalkanol der allgemeinen Formel I

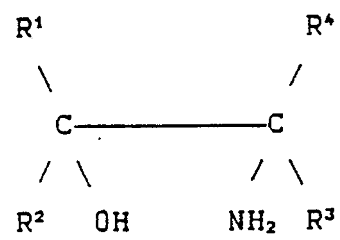

I

worin $R^1$, $R^2$, $R^3$ und $R^4$ obige Bedeutung besitzen, aus dem Reaktionsgemisch isoliert.

Nach dem erfindungsgemäßen Verfahren können Alkylenoxide umgesetzt werden, welche frei von mit Ammoniak reagierenden Substituenten sind und welche in wäßriger mindestens 20 %-iger Ammoniaklösung ausreichend löslich und/oder dispergierbar sind. Um in der wäßrigen Ammoniaklösung ausreichend fein dispergierbar zu sein, müssen in der wäßrigen Ammoniaklösung nicht oder nur schwer lösliche Alkylenoxide bei Temperaturen ab 25 °C flüssig oder gasförmig sein.

Das Verfahren eignet sich beispielsweise zum Umsetzen solcher in mindestens 20 gew.-%-iger wäßriger Ammoniaklösung löslicher oder dispergierbarer Alkylenoxide, worin
$R^1$ eine Alkylgruppe mit 1-10 Kohlenstoffatomen, welche gegebenenfalls durch Hydroxy, niederes Alkoxy, Phenyloxy, Halogen, Trifluormethyl oder Phenyl substituiert ist, oder eine carbocyclische Arylgruppe, welche gegebenenfalls durch niederes Alkyl, niederes Alkoxy oder Halogen substituiert ist, bedeutet,
$R^2$ Wasserstoff oder niederes Alkyl bedeutet,
$R^3$ Wasserstoff oder niederes Alkyl bedeutet, und
$R^4$ Wasserstoff oder niederes Alkyl bedeutet, oder
$R^4$ gemeinsam mit $R^1$ eine Alkylenkette mit 2-10 Kohlenstoffatomen bildet.

Sofern die Reste $R^1$ bis $R^4$ niederes Alkyl darstellen, und sofern $R^1$ niedere Alkyl- oder Alkoxysubstituenten enthält, können diese Gruppen geradkettig oder verzweigt sein und vorzugsweise 1 bis 4 Kohlenstoffatome enthalten.

Die Substituenten $R^2$ und $R^3$ sowie $R^4$, falls dieses nicht zusammen mit $R^1$ eine Alkylengruppe bildet, stellen vorzugsweise Wasserstoff dar. Falls die Reste $R^2$, $R^3$ und/oder $R^4$ niederes Alkyl bedeuten, ist Methyl bevorzugt.

Ein Alkylrest $R^1$ kann ein geradkettiger, verzweigter oder cyclischer Alkylrest sein und beispielsweise bis zu 10, vorzugsweise bis zu 6, Kohlenstoffatome enthalten und gegebenenfalls mit einem der vorstehend angegebenen Substituenten, vorzugsweise Hydroxy, niederes Alkoxy oder Halogen substituiert sein. Als Halogensubstituenten kommen Chlor, Fluor oder Brom in Frage. Vorzugsweise stellt $R^1$ einen niederen Alkylrest, insbesondere Methyl oder Äthyl, dar, welcher unsubstituiert ist oder durch Hydroxy, Methoxy, Äthoxy oder Halogen, vorzugsweise in α-Stellung substituiert ist. Als besonders bevorzugte gegebenenfalls substituierte Alkylreste $R^1$ seien Methyl, Hydroxymethyl, Äthyl und Hydroxyäthyl genannt.

Sofern $R^1$ eine carbocyclische Arylgruppe darstellt, ist diese vorzugsweise Phenyl, welches gegebenenfalls durch die vorstehend genannten Substituenten substituiert sein kann. So kann beispielsweise Styroloxid nach dem erfindungsgemäßen Verfahren zu 1-Hydroxy-2-phenyläthylamin umgesetzt werden.

$R^1$ und $R^4$ können gemeinsam eine Alkylenkette bilden und so zusammen mit den beiden Kohlenstoffatomen, an welche sie gebunden sind, einen carbocyclischen Rest darstellen. Eine aus $R^1$ und $R^4$ gemeinsam gebildete Alkylenkette kann geradkettig oder verzweigt sein und beispielsweise bis zu 10 Kohlenstoffatome, insbesondere bis zu 4 Kohlenstoffatome, enthalten. So kann beispielsweise Cyclohexenoxid nach dem erfindungsgemäßen Verfahren zu 1-Amino-2-hydroxycyclohexan umgesetzt werden.

Insbesondere eignet sich das erfindungsgemäße Verfahren zur Umsetzung von Alkylenoxiden der Formel I, worin $R^1$ Methyl, Äthyl, Hydroxymethyl oder α-Hydroxyäthyl und $R^2$, $R^3$ und $R^4$ je Wasserstoff darstellen, So ist das erfindungsgemäße Verfahren besonders vorteilhaft zur Umsetzung von 2,3-Epoxypropan-1-ol (= Glycidol) zu 1-Aminopropan-2,3-diol.

Erfindungsgemäß wird das Alkylenoxid in Gegenwart einer mindestens äquimolaren Menge eines

Ammoniumsalzes mit mindestens 20 %-iger wäßriger Ammoniaklösung ohne Verwendung von Druck umgesetzt. Zweckmäßig werden im Handel erhältliche konzentrierte wäßrige Ammoniaklösungen mit einem Ammoniakgehalt zwischen 25 und 28 Gew.-% verwendet.

Nach dem erfindungsgemäßen Verfahren der Umsetzung von Alkylenoxiden mit Ammoniak in wäßriger Lösung in Gegenwart einer dem Alkylenoxid mindestens äquivalenten Menge eines Ammoniumsalzes werden zur Bildung von α-Aminoalkanolen das Alkylenoxid und Ammoniak im molaren Verhältnis von 1 : 7 -30, insbesondere 1 : 7 - 25, bevorzugt 1 : 15 - 20, eingesetzt. Das heißt, es wird nur etwa 1/5 bis 1/4 der nach den vorbekannten Verfahren eingesetzten Mengen an wäßriger Ammoniaklösung benötigt.

Als erfindungsgemäß verwendbare Ammoniumsalze eignen sich in wäßriger mindestens 20 %-iger Ammoniaklösung ausreichend lösliche anorganische Ammoniumsalze, insbesondere Halogenide oder Nitrat, oder auch Ammoniumsalze von Halogenessigsäuren wie Chloressigsäure, Trichloressigsäure oder Trifluoessigsäure. Zweckmäßig werden solche Ammoniumsalze eingesetzt, deren Anionen mit Alkalimetallen in niederen Alkoholen schwer lösliche Salze bilden, um so eine einfache spätere Abtrennung aus dem Reaktionsgemisch zu ermöglichen. Als günstig erweisen sich Ammoniumhalogenide. Vorzugsweise werden das Chlorid und/oder das Fluorid eingesetzt, es können jedoch auch Jodid oder Bromid verwendet werden.

Es werden mindestens dem Alkylenoxid äquimolare Mengen an Ammoniumsalz eingesetzt. Beispielsweise können ca. 1 bis 2, insbesondere 1 bis 1,2 Mol Ammoniumsalz pro Mol Alkylenoxid verwendet werden. Größere Überschüsse beeinflussen die Reaktion nicht. Die Auswahl des zu verwendenden Ammoniumsalzes richtet sich nach dem Verdünnungsgrad des Alkylenoxids in der wäßrigen Ammoniaklösung und nach der Löslichkeit des Ammoniumsalzes. Für Reaktionsgemische, welche relativ geringe Mengen Alkylenoxid enthalten, wird beispielsweise Ammoniumfluorid bevorzugt, während für höhere Alkylenoxidkonzentrationen enthaltende Reaktionsgemische das etwas besser lösliche Ammoniumchlorid geeigneter ist.

Die Reaktion kann bei Temperaturen zwischen -20 und 50 °C, insbesondere zwischen -20 und 30 °C, durchgeführt werden. Bevorzugt sind Temperaturen im Bereich zwischen 0 und 30 °C, insbesondere Raumtemperatur.

Zur Durchführung des Verfahrens wird das Ammoniumsalz in der wäßrigen Ammoniaklösung gelöst und das Alkylenoxid in die gewünschtenfalls vorgekühlte das Ammoniumsalz enthaltende wäßrige Ammoniaklösung eingegeben, wobei die Eingabegeschwindigkeit und eine allfällige Kühlung so einreguliert werden, daß die Temperatur im Reaktionsgefäß 50 °C, vorzugsweise 30 °C, nicht überschreitet. Bei Raumtemperatur flüssige Alkylenoxide können über einen Tropftrichter mit entsprechend regulierter Eintropfgeschwindigkeit in den Reaktor eindosiert werden. Bei Raumtemperatur gasförmige Alkylenoxide können beispielsweise aus einer Gasbombe über ein Tauchrohr und eine Gasfritte in die wäßrige Ammoniaklösung eindosiert werden. Vorzugsweise wird die wäßrige Ammoniaklösung auf Temperaturen vorgekühlt, bei denen das einzuführende Alkylenoxid noch flüssig ist, und wird vorzugsweise während der gesamten Eingabezeit auf Temperaturen unter dem Siedepunkt des Alkylenoxids gehalten. Die Eindosierung des Alkylenoxids in die das Ammoniumsalz gelöst enthaltende wäßrige Ammoniaklösung kann gewünschtenfalls unter Rühren erfolgen. Die Eindosiergeschwindigkeit kann so einreguliert sein, daß für die Eindosierung mehrere Stunden, beispielsweise zwischen 3 und 8 Stunden, benötigt werden. Anschließend wird das Reaktionsgemisch zur Vervollständigung der Reaktion mehrere Stunden, beispielsweise bis zu 40 Stunden, vorzugsweise zwischen 15 und 40 Stunden, stehengelassen. Während dieser Zeit des Ausreagierens wird keine weitere Kühlung benötigt, und auch Rühren ist nur in solchen Fällen zweckmäßig, wo das Alkylenoxid in der wäßrigen Ammoniaklösung nicht löslich ist.

Gewünschtenfalls können dem Reaktionsgemisch zur Verbesserung der Löslichkeit des Alkylenoxides geringe Menge eines mit Wasser mischbaren organischen Lösungsmittels, beispielsweise eines niederen Alkoholes, Aceton oder eines cyclischen Äthers wie Tetrahydrofuran zugesetzt werden. Die zugesetzten Mengen sollten jedoch so niedrig sein, daß die Löslichkeit des Ammoniumsalzes und des Ammoniaks im Reaktionsmedium nicht beeinträchtigt werden. Zweckmäßig sollten Zusätze von organischen Lösungsmitteln deshalb 5 Vol.-% bezogen auf die wäßrige Ammoniaklösung nicht überschreiten.

Zur Aufarbeitung des Reaktionsgemisches und Isolierung des gebildeten α-Aminoalkanols wird zweckmäßigerweise zunächst der überschüssige Ammoniak durch Destillation weitgehend aus dem Reaktionsgemisch entfernt, dieses anschließend durch Zugabe von einer mindestens der zugesetzten Ammoniumsalzmenge äquivalenten Menge eines Erdalkali- oder Alkalimetallhydroxides, beispielsweise Natrium- oder Kaliumhydroxid, neutralisiert. Das Hydroxid wird zweckmäßig als solches oder auch in Form einer wäßrigen Hydroxidlösung zugegeben. Anschließend wird so viel Wasser abdestilliert, bis die Hauptmenge des aus dem Anion des Ammonium salzes und dem Erdalkali- oder Alkalimetallkation gebildeten Salzes auskristallisiert ist. Sodann wird der gebildeten Suspension zur Vervollständigung der Salzfällung ein mit Wasser mischbares organisches Lösungsmittel, in welchem das Salz schwer löslich ist, beispielsweise ein niederer Alkohol wie Methanol, Äthanol oder Isopropanol oder auch Aceton, zugesetzt und von dem ausgefällten

Salz abfiltriert. Aus der erhaltenen Lösung kann das α-Aminoalkanol auf an sich bekannte Weise isoliert werden, beispielsweise durch destillative Abtrennung. Das Abtrennen des Ammoniaks aus dem Reaktionsgemisch vor Zugabe des Metallhydroxids kann bei Normaldruck oder vermindertem Druck bei Temperaturen bis zu 50 °C, beispielsweise Temperaturen zwischen 30 und 50 °C, erfolgen. Gewünschtenfalls kann der abdestillierte Ammoniak in einer Kühlfalle aufgefangen und zur Herstellung von in dem Verfahren einsetzbarer wäßrige Ammoniaklösung wieder verwendet werden. Das Abdestillieren des Wassers nach erfolgter Neutralisation kann vorteilhaft unter vermindertem Druck bei erhöhter Temperatur, beispielsweise Temperaturen bis zu 80 °C, durchgeführt werden.

Die Alkylenoxide der Formel II sind bekannt und/oder können nach an sich bekannten oder analog zu an sich bekannten Herstellungsmethoden erhalten werden.

Die nachfolgenden Beispiele sollen die Erfindung näher erläutern, ohne ihren Umfang zu beschränken:

Beispiel 1

Herstellung von 1-Aminopropan-2,3-diol.

In einen 10 l-Dreihalskolben mit Magnetrührer, Rückflußkühler, Tauchthermometer und Tropftrichter ohne Druckausgleich wurden 4,0 l (= 3,64 kg) konzentrierter wäßriger Ammoniaklösung (25 % = 53,5 Mol $NH_3$ ) gegeben. Unter Rühren wurden darin 111 g Ammoniumfluorid (= 3 Mol) gelöst. Anschließend wurden 222 g Glycidol (= 3 Mol) in einem Zeitraum von 7 bis 8 Stunden zu der Lösung getropft. Dabei stieg die Temperatur des Reaktionsgemisches von 20 °C auf 30 °C. Nach Beendigung der Glycidolzugabe wurde die Rührung abgestellt und das Reaktionsgemisch 32 Stunden bei Raumtemperatur stehengelassen.

Zur Aufarbeitung des das gebildete 1-Aminopropan-2, 3-diol enthaltenden Reaktionsgemisches wurde das Gemisch anschließend im Wasserbad bei einer Badtemperatur von 50 °C erwärmt und der Ammoniak bei Normaldruck abdestilliert und in einer auf -40 °C abgekühlten Falle aufgefangen. Nach Beendigung der Ammoniakdestillation wurde das Reaktionsgemisch durch Zugabe von 168,3 g Kaliumhydroxid (= 3 Mol) neutralisiert, und anschließend wurde mit dem Abdestillieren des Wassers bei vermindertem Druck (200 mbar) bei 80 °C im Wasserbad begonnen. Sobald die Hauptmenge an Kaliumfluorid auskristallisiert war, fügte man 200 ml Methanol zu der Suspension, rührte, ließ den ausgefällten Feststoff wieder absitzen und saugte die überstehende Flüssigkeit ab. Dieser Vorgang wurde zweimal wiederholt. Das feste Kaliumfluorid wurde abgetrennt. Die erhaltene methanolische Lösung des 1-Aminopropan-2,3-diols wurde eingeengt, und anschließend wurde das 1-Aminopropan-2,3-diol bei einem Druck von 10 Torr und einer Heizbadtemperatur von 160 °C über einen Liebig-Kühler abdestilliert. Der Siedepunkt bei 10 Torr war 125 °C. Es wurden 198 g 1-Aminopropan-2, 3-diol erhalten (= Ausbeute von 72,5 %).

Die Reinheit des erhaltenen 1-Aminopropan-2,3-diols wurde durch Titration mit 0,1 n-Salzsäure überprüft. Zur Neutralisation von 100 mg 1-Aminopropan-2,3-diol werden theoretisch 10,98 ml 0,1 n-Salzsäure benötigt. Für die Neutralisation von 100 mg des in Beispiel 1 erhaltenen 1-Aminopropan-2, 3-diols wurden 10,90 ml 0,1 n-Salzsäure verbraucht. Dies entspricht einer Reinheit von 99,2 %.

Nach der in Beispiel 1 beschriebenen Methode wurden auch die in der nachstehenden Tabelle I angegebenen Alkylenoxide der Formel II mit Ammoniak unter den in der Tabelle angegebenen Reaktionsbedingungen zu den entsprechenden 1-Aminoalkanolen der Formel I umgesetzt.

TABELLE 1

| Bsp. Nr. | Alkylenoxid II (eingesetzte Menge in g) | Reaktionsbedingungen Mol-Verhältnis II : $NH_3$ | $NH_4$-Salz g | Eingabe t in h Temp. in °C | Nachreaktion t in h Temp. in °C | α-Aminoalkanol (Ausbeute in %) |
|---|---|---|---|---|---|---|
| 2 | Styroloxid (12,2 g) | 1 : 19 | $NH_4F$ 3,7 g | 8 h 25 °C | 40 h 20 °C | 1-Hydroxy-2-phenyl-äthylamin (41,6 %) |
| 3 | Cyclohexenoxid (19,63 g) | 1 : 19 | $NH_4F$ 7,4 g | 8 h 22 °C | 36 h 20 °C | 1-Amino-2-hydroxy-cyclohexan (32,5 %) |
| 4 | Propenoxid (17,42 g) | 1 : 19 | $NH_4F$ 11,1 g | 8 h 30 °C | 40 h 22 °C | 1-Aminopropan-2-ol (47,0 %) |
| 5 | Glycidol (37 g) | 1 : 19 | $NH_4Cl$ 26,5 g | 8 h 30 °C | 32 h 22 °C | 1-Aminopropan-2,3-diol (61,0 %) |

TABELLE 1 (Fortsetzung)

| Bsp. Nr. | Alkylenoxid II (eingesetzte Menge in g) | Reaktionsbedingungen Mol-Verhältnis II: $NH_3$ | $NH_4$-Salz g | Eingabe t in h / Temp. in °C | Nachreaktion t in h / Temp. in °C | α-Aminoalkanol (Ausbeute in %) |
|---|---|---|---|---|---|---|
| 6 | Glycidol (37 g) | 1 : 13 | $NH_4F$ 18,5 g | 8 h / 30 °C | 32 h / 22 °C | 1-Aminopropan-2,3-diol (67,0 %) |
| 7 | Glycidol (37 g) | 1 : 8 | $NH_4Cl$ 26,5 g | 8 h / 30 °C | 32 h / 22 °C | 1-Amipropan-2,3-diol (56,0 %) |
| 8 | Glycidol (37 g) | 1 : 10 | $NH_4F$ 18,5 g | 8 h / 30 °C | 32 h / 20 °C | 1-Aminopropan-2,3-diol (60,5 %) |
| 9 | Glycidol (37 g) | 1 : 25 | $NH_4F$ 18,5 g | 8 h / 30 °C | 20 h / 20 °C | 1-Aminopropan-2,3-diol (73,5 %) |

**Ansprüche**

1. Verfahren zur Herstellung von α-Aminoalkanolen durch Umsetzen von Alkylenoxiden mit wäßriger Ammoniaklösung, dadurch gekennzeichnet, daß man
in mindestens 20 Gew.-%-iger wäßriger Ammoniaklösung lösliche oder dispergierbare Alkylenoxide der allgemeinen Formel II

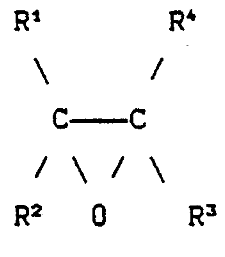

II

worin
$R^1$ Wasserstoff oder einen aliphatischen oder aromatischen Rest bedeutet,
$R^2$ Wasserstoff oder niederes Alkyl bedeutet,
$R^3$ Wasserstoff oder niederes Alkyl bedeutet, und
$R^4$ Wasserstoff oder niederes Alkyl bedeutet, oder
$R^4$ und $R^1$ gemeinsam mit den beiden Kohlenstoffatomen, an welche sie gebunden sind, eine carbocyclische Gruppe bilden,
mit mindestens 20 Gew.-%-iger wäßriger Ammoniaklösung in Gegenwart einer dem Alkylenoxid mindestens äquimolaren Menge eines in der mindestens 20 gew.-%-igen wäßrigen Ammoniaklösung löslichen Ammoniumsalzes umsetzt
und das gebildete α-Aminoalkanol der allgemeinen Formel I

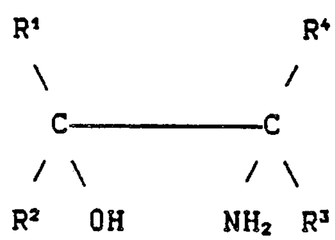

I

worin $R^1$, $R^2$, $R^3$ und $R^4$ obige Bedeutung besitzen, aus dem Reaktionsgemisch isoliert.

2. Verfahren nach Anspruch 1, dadurch gekennzeichnet, daß Alkylenoxide umgesetzt werden, worin
$R^1$ eine Alkylgruppe mit 1-10 Kohlenstoffatomen, welche gegebenenfalls durch Hydroxy, niederes Alkoxy, Phenyloxy, Halogen, Trifluormethyl oder Phenyl substituiert ist, oder eine carbocyclische Arylgruppe, welche gegebenenfalls durch niederes Alkyl, niederes Alkoxy oder Halogen substituiert ist, bedeutet,
$R^2$ Wasserstoff oder niederes Alkyl bedeutet,
$R^3$ Wasserstoff oder niederes Alkyl bedeutet, und
$R^4$ Wasserstoff oder niederes Alkyl bedeutet, oder
$R^4$ gemeinsam mit $R^1$ eine Alkylenkette mit 2-10 Kohlenstoffatomen bildet.

3. Verfahren nach Anspruch 2, dadurch gekennzeichnet, daß man solche Alkylenoxide einsetzt, worin $R^1$ ein gegebenenfalls durch Hydroxy oder niederes Alkoxy substituiertes niederes Alkyl bedeutet und $R^2$, $R^3$ und $R^4$ Wasserstoff bedeuten.

4. Verfahren nach Anspruch 3, dadurch gekennzeichnet, daß man als Alkylenoxid 2,3-Epoxypropan-1-ol einsetzt.

5. Verfahren nach einem der vorhergehenden Ansprüche, dadurch gekennzeichnet, daß man ein molares Verhältnis von Alkylenoxid zu Ammoniak von 1 : 7 - 30, insbesondere 1 : 10 - 25, einsetzt.

6. Verfahren nach Anspruch 3, dadurch gekennzeichnet, daß man ein molares Verhältnis von Alkylenoxid zu Ammoniak von 1 : 15 - 20 einsetzt.

7. Verfahren nach einem der vorhergehenden Ansprüche, dadurch gekennzeichnet, daß man als Ammoniumsalz ein in mindestens 20 gew.-%-iger Ammoniaklösung lösliches Ammoniumsalz einer anorganischen Säure oder einer Halogenessigsäure einsetzt.

8. Verfahren nach Anspruch 7, dadurch gekennzeichnet, daß man als Ammoniumsalze Ammoniumhalogenide, insbesondere Fluorid oder Chlorid einsetzt.

9. Verfahren nach einem der vorhergehenden Ansprüche, dadurch gekennzeichnet, daß die Umsetzung bei Temperaturen zwischen -20 und 50 °C, insbesondere zwischen -20 und 30 °C, erfolgt.

10. Verfahren nach Anspruch 1, dadurch gekennzeichnet, daß zur Isolierung des α-Aminoalkanols aus dem Reaktionsgemisch zunächst Ammoniak abdestilliert wird, das verbleibende Reaktionsgemisch mit einem Erdalkali- oder Alkalimetallhydroxid neutralisiert wird, und das aus dem Erdalkali- oder Alkalimetallkation und dem Anion des Ammoniumsalzes gebildete Salz durch Einengen der Lösung und Zugabe eines mit Wasser mischbaren organischen Lösungsmittels ausgefällt wird und das α-Aminoalkanol aus der verbleibenden Lösung isoliert wird.

Europäisches Patentamt

# EUROPÄISCHER RECHERCHENBERICHT

Nummer der Anmeldung

EP 89 11 6165

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-C- 694 992 (THE GIRDLER CORP.) * das ganze Dokument * --- | 1,2,5,7-10 | C 07 C 213/04<br>C 07 C 215/08<br>C 07 C 215/10<br>C 07 C 215/42 |
| X | US-A-2 186 392 (A.F.A. REYNHART et al.) * Seite 3, Beispiel 1; Ansprüche 1-3 * | 1,5-7,9 | |
| Y | --- | 1,5-10 | |
| Y | JOURNAL OF THE AMERICAN CHEMICAL SOCIETY Band, 72, Nr. 9, 16. September 1950, Seiten 4669-4667, American Chemical Society, Washington, D.C., USA; S. WINSTEIN et al.: "The role of the benzamido group in replacement reactions" * Seite 4675, Spalte 1 * --- | 1,5-10 | |
| A | EP-A-0 171 893 (BP CHEMICAL LTD.) * Seite 1, Linie 24; Ansprüche 1-3,6,8 * --- | 1-3,5,7,9 | |
| | | | RECHERCHIERTE SACHGEBIETE (Int. Cl.5) |
| A | EP-A-0 037 889 (DEGUSSA AG) * Seiten 5,6; Ansprüche 1,2,5-7 *; & DE - A - 3014129 (Cat. D) --- | 1-6 | C 07 C 213/00<br>C 07 C 215/00 |
| A | EP-A-0 037 890 (DEGUSSA AG) * Seiten 4-6 *; & DE - A - 30 14098 (Cat. D) ----- | 1-6 | |

Der vorliegende Recherchenbericht wurde für alle Patentansprüche erstellt

| Recherchenort | Abschlußdatum der Recherche | Prüfer |
|---|---|---|
| BERLIN | 15-01-1990 | RUFET J.M.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument